# EUROPEAN PATENT APPLICATION

(11) **EP 4 152 037 A1**
(43) Date of publication of application: **22.03.2023**
(21) Application number: 21197259.1
(22) Date of filing: 16.09.2021
(51) Int. Cl.: G01R 33/563, A61B 5/026, A61B 6/00, A61B 5/00

(54) **APPARATUS AND METHOD FOR GENERATING A PERFUSION IMAGE, AND METHOD FOR TRAINING AN ARTIFICIAL NEURAL NETWORK THEREFOR**

(71) Applicant: Friedrich-Alexander-Universität Erlangen-Nürnberg, 91054 Erlangen (DE)
(72) Inventor: Bickelhaupt, Sebastian, 91054 Erlangen (DE); Liebert, Andrzej, 91054 Erlangen (DE); Schreiter, Hannes, 91054 Erlangen (DE); Sukumar, Vishal, 91054 Erlangen (DE)
(74) Representative: Isarpatent

(57) **Abstract**

The invention provides an apparatus and a method for generating a perfusion image, as well as a method for training an artificial neural network for use therein.

The method comprises at least steps of:
receiving (S100) at least one non-contrast medical diagnostic image, NCMDI (1-i), acquired from organic tissue;
generating (S200), using an artificial neural network, ANN (2), trained and configured to receive input data (10) based on at least one of the received at least one non-contrast medical diagnostic image, NCMDI (1-i), based on the input data (10), at least a perfusion image (3) for the organic tissue shown in the at least one non-contrast medical diagnostic image, NCMDI (1-i); and
outputting (S300) at least the generated perfusion image (3).

## Description

### Field of the Invention

The invention relates to the field of medical diagnostic imaging. Within the field of medical diagnostic imaging, the invention relates preferably to magnetic resonance imaging, MRI. Some aspects of the invention relate to an apparatus or a method that allow extracting and visualizing perfusion characteristics of a tissue or organ without the application of contrast agents. Some aspects relate to the training of an artificial neural network or machine learning technique usable in such an apparatus or a method.

### Background of the Invention

Medical imaging techniques are increasingly used for medical diagnostics, in part because advances in medical image analysis by computer systems, in particular artificial intelligence entities have made the analysis of medical diagnostic images significantly cheaper and more accessible.

In many medical diagnostic tasks, a so-called perfusion image is required. A perfusion image comprises, or indicates, information about the passage of fluid through living tissue, for example the passage of blood through elements of the circulatory system, or the passage of lymphatic fluid through the lymphatic system.

The currently known methods for producing a perfusion image are quite involved and demand considerable resources: first, a non-contrast magnetic resonance imaging, MRI, image is acquired using an MRI scanner. Then, a contrast-enhanced MRI image is acquired, preferably using the same MRI scanner, wherein the position and orientation of the patient should ideally be as similar as possible. "Non-contrast" in this context means that no (or essentially no) MRI contrast agent is administered to the patient before or during the acquisition of the respective MRI data (or: exam, or: scan) such that only the response by the living tissue, and no additional response by any MRI contrast agent, is acquired in the non-contrast MRI image. By comparison, a "contrast-enhanced" MRI image is an MRI image which is acquired while an amount of MRI contrast agent is present in the patient, or specifically in the living tissue to be scanned. "Non-contrast" images are sometimes also designated as "Non-contrast-enhanced" instead.

The two acquired images are then co-registered and optionally further processed in order to generate a so-called "subtraction image" which is a perfusion only depiction of tissue. The term "subtraction" indicates that the information in this kind of perfusion image is generated by "subtracting" the information in the non-contrast MRI image from the contrast-enhanced MRI image. In this way, the bulk of the remaining information is due to the perfusion of the contrast agent and thus indicates a perfusion pattern or, if acquisitions are repeated over time, perfusion dynamics within the living tissue.

MRI contrast agents carry an inherent potential for adverse effects, e.g. but not exclusively for allergic reactions up to anaphylactic reactions and a more recently described potential to cause depositions in the human body. Further the administration of the MRI contrast agents cause a substantial periprocedural effort necessary.

The use of convolutional neural networks with specific architecture for biomedical image segmentation is known for example from the scientific publication of Ronneberger et al., "U-Net: Convolutional networks for biomedical image segmentation", Medical Image Computing and Computer-Assisted Intervention (MICCAI), Springer, LMCS, Vol. 9351: 234-241, 2015, which will in the following be cited as "Ronneberger et al.". In short, the U-net is a network comprising convolutional layers wherein a first section of the U-net is an encoder branch followed by a usually (essentially) equally-sized decoder branch. Similar in principle to an autoencoder, from biomedical images first, in the encoder branch, highly relevant information-rich hidden layers are generated which are then, in the decoder branch, decoded again in order to generate segmented medical images.

The U-net receives its name by one or more shortcut connections between parts of the encoder branch and the decoder branch in which hidden features are transferred from the encoder branch to the decoder branch. This means that in the decoder branch not only the highly encoded end product of the encoder branch is used, but also comparatively lightly encoded previous hidden features of the encoder branch which may retain more of the original structural information about the input image. The shortcut connections are usually drawn as straight lines such that the encoder branch and the decoder branch are depicted as the two essentially symmetric sides of a "U-shape" with the shortcut connections arranged in between.

Convolutional neural networks of the U-net type have achieved considerable success in biomedical image segmentation. Segmentation is the task of providing masks for images, the masks indicating for each pixel of the image to which of a predefined set of classes it belongs. Different classes in the field of biomedical image segmentation usually indicate different types of organic tissue, with one class being reserved for the image background.

### Summary of the invention

One of the objectives of the present invention is to provide an improved system and an improved method for obtaining perfusion images without the necessity of the administration of contrast agents. Another objective is to train an artificial neural network effectively and efficiently for use in such a system or method. These objectives - among others - are solved by the subject matter of the independent claims.

Accordingly, according to a first aspect of the present invention, an apparatus for generating a perfusion image is provided, the apparatus comprising:
an input module (or: input interface) configured to receive at least one non-contrast medical diagnostic image, NCMDI, acquired from (or: showing, or: depicting, or: comprising) organic tissue; a computing device configured to implement an artificial neural network, ANN, which is trained and configured to receive input data based on at least one of the received at least one non-contrast medical diagnostic image, NCMDI, and to generate, based on the input data, a perfusion image for the living organic tissue in the at least one non-contrast medical diagnostic image, NCMDI; and
an output module (or: output interface) configured to output at least the generated perfusion image.

The system according to the present invention results in less imaging scans being necessary, in particular less contrast-enhanced imaging scans which always put some degree of stress on the body of a patient. Moreover, less processing steps are necessary, as in particular the steps of calculating the subtraction image become unnecessary. Both of these advantages also lead to another advantage, which is the significant reduction in potential artifact sources. Since artifacts can occur in each medical imaging scan, and again in many mathematical operations, in particular when subtractions are involved, the reduction of both scans and calculation steps reduces the number of artifacts.

In other words, the apparatus according to embodiments of the first aspect of the present invention functions in the inference stage, i.e. when it is applied to previously unseen non-contrast medical diagnostic images, NCMIs, without any contrast-enhanced medical diagnostic image, CEMDI, of the patient under examination being necessary. While such CEMDIs may be necessary in some variants for the training (i.e. during the training stage) of the artificial neural network, ANN, these CEMDIs need not (and will usually not) be of the patient under examination in the inference stage. Accordingly, the apparatus and methods according to the present invention can be used for generating perfusion diagnostics data for a patient (a perfusion image, perfusion dynamics data etc.) without the necessity to administer a contrast agent to the patient. This not only reduces the stress on the body of the patient but also allows diagnosis of patients to whom a contrast agent cannot be administered for whatever reason. Coincidentally, resources for producing contrast agents are spared.

Another one of the main ideas of the present invention is that the image that is generated is not a virtual version of an actual acquisition image (which may then be processed further) but the perfusion image which cannot be acquired directly at all.

As a side note, the term "image" as used herein shall not necessarily indicate a human-interpretable picture such as a bitmap but may also refer to acquisition data in general. For example, in magnetic resonance imaging, MRI, the acquired data are magnetic field strength data and time data, from which then a human-interpretable picture can be generated. The term "image" is herein therefore used synonymously with "imaging result".

The organic tissue is preferably living organic tissue, preferably living organic tissue, more preferably living mammalian organic tissue, most preferably living human organic tissue.

Preferably, a plurality of non-contrast medical diagnostic images, NCMDIs, is used as input data on which the perfusion image is then generated by the artificial neural network, ANN. In another embodiment the plurality of NCMDIs used as input data for the ANN allows to generate not only a solitary perfusion image, but for generating so-called dynamic perfusion images depicting the dynamic component of the perfusion of a tissue. The assessment of the dynamic perfusion component of a tissue is routinely performed by the administration of a contrast agent that then allows to observe and/or (semi-)quantitatively assess the perfusion pattern of the contrast agent within the tissue. Commonly used terms for this include the "wash-in" and/or "plateau" and/or "wash-out" phases of the perfusion dynamics. However other terms and characterization methods might be used by those familiar with the art and further characterizations techniques might be applied, e.g. but not exclusively by assessing the steepness or relative or absolute increase of signal intensity in an image due to the arrival of contrast agent in the tissue.

The herein presented invention allows extracting those perfusion dynamic characteristics out of NCMDIs using an ANN. For example, three or more, four or more, five or more, or still more individual time points of a perfusion characteristic might be generated by the input of NCMDIs into the ANN. It will be understood that in these contexts, when the input data are discussed, it is preferred that the NCMDIs all stem from the same patient, and more preferred that they stem from the same examination session, i.e. are all acquired within a time frame as known to those familiar to the art of MR imaging.

It is one of the surprising findings of the inventors of the present invention that even a single NCMDI taken at one point in time is sufficient - for an ANN trained as described herein - to generate a series of accurate predictions for perfusion images over several points in time. It turns out that the ANN configured and trained as described herein is capable of such extraordinary predictions.

The input data may comprise, or consist of, at least one of the received at least one non-contrast medical diagnostic image, NCMDI, and may comprise, or consist of, all of the received at least one non-contrast medical diagnostic images, NCMDIs, for example all of a plurality of received non-contrast medical diagnostic images, NCMDIs. Alternatively, the input data may be derived from at least one of the received at least one non-contrast medical diagnostic image, NCMDI. For example, any received non-contrast medical diagnostic image, NCMDI, may be subjected to one or more preprocessing steps.

The at least one non-contrast medical diagnostic image, NCMDI, may in particular be the acquisition result of a diffusion-weighted magnetic resonance imaging, MRI, sequence, or, in short, an acquisition result of diffusion-weighted imaging, DWI, preferably with multiple b-values. However, the at least one non-contrast medical diagnostic image, NCMDI, may, alternatively or additionally, also comprise, or consist of, acquisition results of a DWI sequence and/or acquisition results of a T1-weighted MRI sequence and/or acquisition results of a T2-weighted MRI sequence. However this is not to be understood as an exclusive list of feasible MRI acquisition techniques to be used for the purpose of the presented invention. Other acquisition techniques known to those skilled in the art, and other techniques yet to emerge might be used as well.

Preprocessing may include, for example, data augmentation, co-registration of several images and/or the like.

The computing device may be realized as any device, or any means, for computing, in particular for executing a software, an App or an algorithm. For example, the computing device may comprise at least one processing unit such as at least one central processing unit, CPU, and/or at least one graphics processing unit, GPU, and/or at least one field-programmable gate array, FPGA, and/or at least one application-specific integrated circuit, ASIC, and/or any combination of the foregoing.

The computing device may further comprise a working memory operatively connected to the at least one processing unit and/or a non-transitory memory operatively connected to the at least one processing unit and/or a working memory. The computing device may be realized as a local device, as a remote device (such as a server connected remotely to a client with a user interface) or as a combination of these. A part, or all, of the computing device may also be implemented by a cloud computing platform. The input module and/or the output module may also be integrated into the computing device.

Although, here, in the foregoing and also in the following, some functions are described as being performed by modules, it shall be understood that this does not necessarily mean that such modules are provided as entities separate from one another. In cases where one or more modules are provided as software, the modules may be implemented by program code sections or program code snippets which may be distinct from one another but which may also be interwoven or integrated into one another.

Similarly, in cases where one or more modules are provided as hardware, the functions of one or more modules may be provided by one and the same hardware component, or the functions of several modules may be distributed over several hardware components which need not necessarily correspond to the modules. Thus, any apparatus, system, method and so on which exhibits all of the features and functions ascribed to a specific module shall be understood to comprise, or implement, said module. In particular, it is a possibility that all modules are implemented by program code executed by the computing device, for example a server or a cloud computing platform.

The artificial neural network, ANN, may in particular be a deep learning, DL, network, specifically a network including a convolutional layer, preferably of the so-called U-net type, see for example the publication by Ronneberger et al. The inventors have discovered that, surprisingly, U-net type deep learning networks (which are usually employed for segmentation tasks) are well suited for the apparatus and methods according to the present invention.

According to a second aspect of the present invention, a computer-implemented method for generating a perfusion image is provided, the method comprising steps of:
- receiving at least one non-contrast medical diagnostic image, NCMDI, acquired from (or: showing, or: depicting, or: comprising) organic tissue (preferably living organic tissue, more preferably living mammalian organic tissue, most preferably living human organic tissue);
- using an artificial neural network, ANN, trained and configured to receive input data based on at least one of the received at least one medical diagnostic image, MDI, generating, based on the input data, at least a perfusion image for the living organic tissue in the at least one medical diagnostic image, MDI; and
- outputting at least the generated perfusion image.

A step of acquiring the at least one non-contrast medical diagnostic image, NCMDI, may also be part of the method. This may be performed by a medical imaging scanner (or: medical imaging device) which may or may not be part of the apparatus according to the present invention.

The embodiments of the second aspect of the present invention can be modified or refined according to any modification, option, variation, or refinement that is described herein with respect to the apparatus according to the first aspect of the present invention.

According to a third aspect of the present invention, a computer-implemented method for training an artificial neural network for generating a perfusion image is provided, the method comprising steps of:
providing a training set of medical diagnostic training image groups, MDTIG, wherein each medical diagnostic training image group, MDTIG, comprises at least:
   - a non-contrast medical diagnostic image, NCMDI; and at least one subtraction image based on the NCMDI;
providing an artificial neural network, ANN, configured to receive, as input data, a non-contrast medical diagnostic image, NCMDI, and to generate, based on the input data, at least a perfusion image;
training the provided artificial neural network using the provided training set of medical diagnostic training image groups, MDTIG, using supervised learning while penalizing differences between the generated perfusion image and at least one of the at least one subtraction image.

In other words, each medical diagnostic training image group, MDTIG, comprises at least a non-contrast medical diagnostic image, NCMDI, (preferably more than one NCMDIs) and a corresponding label for the training. The label is given by the at least one subtraction image and indicates the desired output of the artificial neural network, ANN, when the corresponding NCMDI of the same MDTIG is input into the ANN. One exemplary method for generating an MDTIG for one patient is the following:
1) an NCMDI may be acquired from a patient,
2) then, a contrast agent sequence is initiated (i.e. contrast agent is administered according to some predetermined sequence,
3) over a time of (for example) 5 minutes, each minute an contrast-enhanced medical diagnostic image, CEMDI, is acquired,
4) based on the NCMDI and at least one of the CEMDIs, a subtraction image is calculated.If this exemplary method is used to train an ANN to generate results for multiple points in time, these points in time may correspond to the points in time depicted by the CEMDI. Thus, for example, if a NCMDI is generated on January 1st, 2020, at 9:15 a.m., then the results of a trained ANN which uses this NCMDI as input may correspond to points in time at 9:16, 9:17, 9:18, 9:19 and 9:20 a.m., respectively.

Preferably, the medical data training image groups, MDTIGs, comprise data from a plurality of patients, and more preferably each medical data training image group, MDTIG, stems from a different patient or at least from a different examination session. In this way, possible overfitting to a small number of patients is avoided, and the artificial neural network, ANN, is better trained to generate perfusion images for a large variety of living organic tissue.

It should be noted that a "difference" in this sense need not necessarily be the result of a simple subtraction operation, although in some case it may be just that. However, a difference may be calculated in any kind of suitable difference metric known in the prior art.

Terms which penalize differences between the generated data of an artificial neural network, ANN, and the corresponding label data (or: ground truth data) are commonly called loss functions. The skilled person is aware of different kinds of loss functions such as mean squared error (MSE) loss functions, sum of squared errors (SSE) loss functions and the like which may be applied. The differences may in particular be calculated or considered pixel-wise, i.e. a loss (or: cost) term is calculated for each pixel based on the difference between the value generated for that pixel and the value given for that pixel by the corresponding label or ground truth.

Since the subtraction images calculated according to the prior art need both an non-contrast medical diagnostic image, NCMDI, and a contrast-enhanced medical diagnostic image, CEMDI, which cannot be taken at the same time (because either a contrast agent is present in the patient or not but not both at the same time), the subtraction image does not come with a specific time stamp. However, it will in each instance be clear with which non-contrast medical diagnostic image, NCMDI, and with which contrast-enhanced medical diagnostic image, CEMDI, a particular subtraction image is associated, name the ones from which it is derived (or: calculated). For the loss function, the perfusion image may in particular be compared to a subtraction image based on (or: derived from) the same NCMDI which has also been used for deriving the subtraction image. It is possible that more than one subtraction image is derived using the same NCMDI but multiple CEMDIs, in particular CEMDIs at different points in time.

According to a fourth aspect, the invention provides a computer program product comprising executable program code configured to, when executed, perform the method according to any embodiment of the second aspect or the third aspect of the present invention.

According to a fifth aspect, the invention provides a non-transient computer-readable data storage medium comprising executable program code configured to, when executed, perform the method according to any embodiment of the second aspect or the third aspect of the present invention.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick, or the like.

According to a sixth aspect, the invention provides a data stream comprising, or configured to generate, executable program code configured to, when executed, perform the method according to any embodiment of the second aspect or the third aspect of the present invention.

Further advantageous variants, embodiments and refinements are found in the dependent claims as well as in the description with reference to the attached drawings.

In some advantageous embodiments, refinements, or variants of embodiments, at least one of the at least one non-contrast medical diagnostic image, NCMDI, is a non-contrast magnetic resonance imaging result, NCMRIR. It has been found by the inventors that the present invention can be applied very successfully on such types of images. Preferably, all of the at least one non-contrast medical diagnostic images, NCMDIs, are non-contrast magnetic resonance imaging results, NCMRIR.

In some advantageous embodiments, refinements, or variants of embodiments, the living organic tissue is breast tissue. Perfusion images for breast diagnostics are an important tool.

Its importance is balanced to some degree by the stress put on the body of the patient by the contrast agent when contrast-enhanced medical diagnostic images, CEMDI, are acquired. The present invention which allows the generation of perfusion images without the use of a contrast agent in the inference stage (or: deployment stage) thus greatly increases the viability of this diagnostic tool.

It shall be understood that the invention is applicable to other kinds of tissue as well, in particular tissue where commonly contrast-enhanced medical diagnostic images, CEMDIs, are acquired and/or where perfusion images are needed for the diagnosis. Another example for such a field is the field of prostate diagnostics. Thus, the medical diagnostic images described herein for the training stage as well as for the inference stage may be medical breast images, medical prostate images and/or the like.

In some variants, the non-contrast medical diagnostic image, NCMDI, may be replaced by a low-contrast medical diagnostic image, LCMDI. Possible definitions for non-contrast medical diagnostic images, NCMDIs, low-contrast medical diagnostic images, LCMDIs, and contrast-enhanced medical diagnostic images, CEMDIs, can be given as follows: NCMDIs refer to image acquisitions that do not include the administration of contrast enhancing agents. CEMDIs refer to image acquisitions that include the administration of contrast enhancing agents in a dosing scheme following the clinical routine standard as e.g. described in the pharmaceutical approval documents, clinical guidelines and other documents suitable to define recommendations and the clinical routine. Such guidelines and recommendations are provided, for example, by publications such as R. Mann et al., Breast MRI: guidelines from the European Societa of Breast Imaging, Eur Radiol. 2008; 18(7):1307-1318, published 2008 Apr 4, doi: 10.1007/s00330-008-0863-7 or such as the ACR PRACTICE PARAMETER FOR THE PERFORMANCE OF CONTRAST-ENHANCED MAGNETIC RESONANCE IMAGING (MRI) OF THE BREAST, Revised 2018 (Resolution 34), of the American College of Radiology, available for instance at https://www.acr.org/- /media/acr/files/practice-parameters/mr-contrast-breast.pdf.

LCMDIs refer to image acquisition including the administration of contrast enhancing agents, however deviating in the dosing scheme from the clinical routine standard.

In some advantageous embodiments, refinements, or variants of embodiments, the input module is configured to receive a plurality of non-contrast medical diagnostic images, NCMDIs, and the artificial neural network, ANN, is configured to receive said plurality as its input data and to generate out of the NCMDIs a plurality of perfusion images depicting different points in time, PIT, of the perfusion pattern observed after a contrast agent administration. This plurality may cover a time span of 5 minutes or any other timespan, and interval. As has been described in the foregoing, this may improve the resulting perfusion image since the plurality comprises information about the perfusion, i.e. about changes over time.

Advantageously, the artificial neural network, ANN, is thus further configured to generate, based on the input data, in addition to the perfusion image also perfusion dynamics data, i.e. dynamic time-resolved perfusion information. This provides a user, for example a physician, with additional valuable information about the state of the patient, again without having to perform any flow measurements or administering any contrast agent. The assessment of the dynamic perfusion component of a tissue is routinely performed by the administration of a contrast agent that then allows to observe and/or (semi-)quantitatively assess the perfusion pattern of the contrast agent within the tissue.

Commonly used terms for this include the "wash-in" and/or "plateau" and/or "wash-out" phases of the perfusion dynamics. However other terms and characterization methods might be used by those skilled in the art and further characterization techniques might be applied, e.g. but not exclusively by assessing the steepness or relative or absolute increase of signal intensity in an image due to the arrival of contrast agent in the tissue. This information can exemplarily, but not exclusively be used to generate a so called "contrast agent curve" depicting the signal intensity change over time after the administration of the contrast agent, which can be analyzed visually and/or (semi-)quantitatively in order to characterize the underlying tissue, since it has been demonstrated that, e.g., malignant lesions can exhibit different perfusion characteristics than benign lesions.

The perfusion dynamics data may be, for example, represented by a set of data point tuples (or a curve) indicating, for example, a perfusion intensity as a function of time. In some variants, the artificial neural network, ANN, may even be configured to such as to generate the perfusion dynamics data instead of the perfusion image. In the latter variants, the system may be designated as a system for generating perfusion dynamics data, the method may be designated as a method for generating perfusion dynamics data, and so on.

In some advantageous embodiments, refinements, or variants of embodiments of the method according to the third aspect of the present invention, each medical data training image group, MDTIG, further comprises at least one contrast-enhanced medical diagnostic image, CEMDI; and
the providing of the training set of MDTIGs comprises calculating the at least one subtraction image of the MDTIG based on the non-contrast medical diagnostic image, NCMDI, and on the at least one contrast-enhanced medical diagnostic image, CEMDI, of the MDTIG.

The calculating (or: deriving) of the subtraction image may be performed according to the methods known in the prior art, i.e. at least by co-registering a corresponding NCMDI and CEMDI and then performing the subtraction. If a plurality of CEMDIs is available, then using a single NCMDI, also a plurality of subtraction images may be calculated.

In some advantageous embodiments, refinements, or variants of embodiments, each medical data training image group, MDTIG, comprises a plurality of non-contrast medical diagnostic images, NCMDIs. The artificial neural network, ANN, may be configured to receive, as input data (i.e. as its input at its input nodes), the plurality of NCMDIs and to generate the perfusion image for the supervised learning based on these input data.

In some advantageous embodiments, refinements, or variants of embodiments, each MDTIG further comprises a perfusion dynamics data label. The artificial neural network, ANN, may be further configured to generate, based on the input data, perfusion dynamics data. The training of the ANN may further comprise penalizing differences between the generated perfusion dynamics data and the perfusion dynamics data label.

### Brief description of the drawings

The invention will be explained in greater detail with reference to exemplary embodiments depicted in the drawings as appended.

The accompanying drawings are included to provide a further understanding of the present invention and are incorporated in and constitute a part of this specification. The drawings illustrate the embodiments of the present invention and together with the description serve to explain the principles of the invention. Other embodiments of the present invention and many of the intended advantages of the present invention will be readily appreciated as they become better understood by reference to the following detailed description. The elements of the drawings are not necessarily to scale relative to each other. Like reference numerals designate corresponding similar parts.

In the figures:
- Fig. 1: shows a schematic block diagram illustrating an apparatus according to an embodiment of the first aspect of the present invention;
- Fig. 2: schematically illustrates one of the basic ideas of the present invention;
- Fig. 3: schematically depicts a possible artificial neural network architecture;
- Fig. 4: shows a schematic flow diagram illustrating a method according to an embodiment of the second aspect of the present invention;
- Fig. 5: shows a schematic flow diagram illustrating a method according to an embodiment of the third aspect of the present invention;
- Fig. 6: shows a schematic block diagram illustrating a computer program product to an embodiment of the fourth aspect of the present invention;
- Fig. 7: shows a schematic block diagram illustrating a computer program product to an embodiment of the fifth aspect of the present invention; and
- Fig. 8: shows comparison data between results of the present invention and results according to the prior art.

### Detailed description of the invention

Although specific embodiments have been illustrated and described herein, it will be appreciated by those of ordinary skill in the art that a variety of alternate and/or equivalent implementations may be substituted for the specific embodiments shown and described without departing from the scope of the present invention. Generally, this application is intended to cover any adaptations or variations of the specific embodiments discussed herein.

In the described examples and in connection with the drawings, as a particularly useful example the living tissue will be human breast tissue, and the non-contrast medical diagnostic images, NCMDIs, will be deemed to be made using non-contrast magnetic resonance imaging. However, it shall be understood that also any other type of living organic tissue for which perfusion images or perfusion information are relevant, may be substituted analogously.

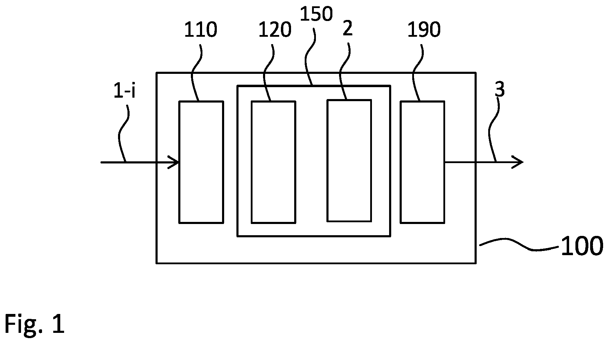

Fig. 1 shows a schematic block diagram illustrating an apparatus 100 according to an embodiment of the first aspect of the present invention, i.e. an apparatus for generating a perfusion image.

The apparatus 100 comprises:
- an input module 110 configured to receive at least one non-contrast medical diagnostic image, NCMDI 1-i, acquired from organic tissue;
- a computing device 150 configured to implement an artificial neural network, ANN 2, which is trained and configured to receive input data based on at least one of the received at least one non-contrast medical diagnostic image, NCMDI, and to generate, based on the input data, at least a perfusion image 3 for the living organic tissue in the at least one non-contrast medical diagnostic image, NCMDI; and
- an output module 190 configured to output at least the generated perfusion image 3.

The generated perfusion image 3 may be output, for example, to a picture archiving and communications system, PACS, for storing, further processing, and/or examination by a physician. The generated perfusion image 3 may also be output directly to a display device 195 which may be a part of the apparatus 100 or which may be separate from it, as exemplarily shown in Fig. 1. When the apparatus 100 comprises, or is part of, a medical imaging device such as an MRI scanner, it is advantageous if the generated perfusion image is displayed essentially in real-time to a user operating the medical imaging device and/or to a physician. The user or physician may thus be enabled to decide whether the perfusion image 3 is sufficient or whether it shows any deficiencies such that another medical image acquisition may be necessary while the patient is still present.

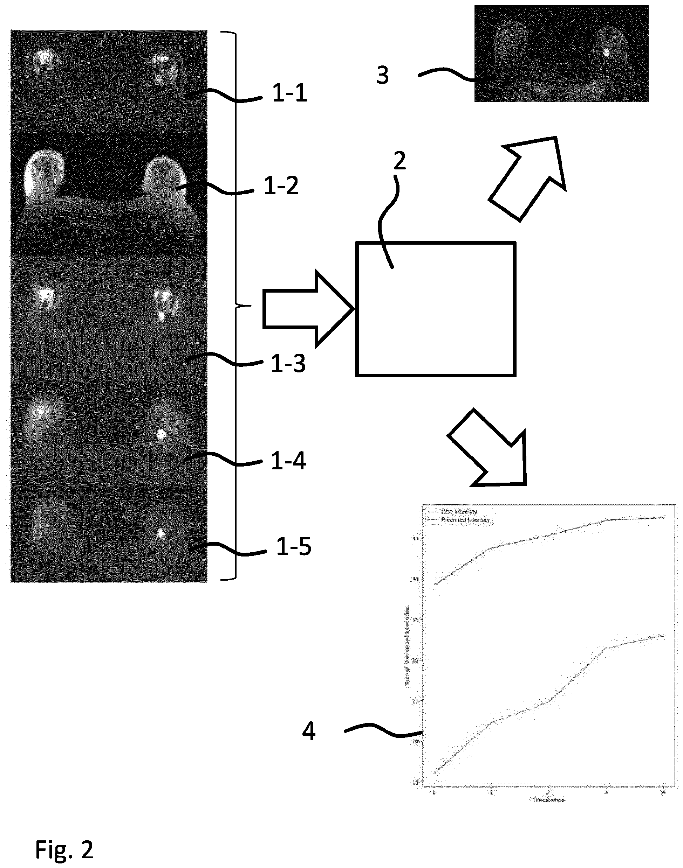

One of the basic ideas of the present invention is shown in Fig. 2: on the left-hand side, a set of five non-contrast medical diagnostic images, NCMDIs 1-1, 1-2, 1-3, 1-4, and 1-5 (hereafter sometimes collectively referred to as 1-i) is shown. Preferably, these NCMDIs 1-i are acquired from (i.e. show) the same patient and are taken at different points in time during the same examination session, for example at regular intervals within a time span of 5 minutes. The NCMDIs 1-i are non-contrast magnetic resonance imaging results, NCMRIR of human breasts, so that the living organic tissue under examination is breast tissue.

Fig. 2 schematically illustrates that the NCMDIs 1-i are input into an artificial neural network, ANN 2, which then generates a perfusion image 3, and optionally also (or even alternatively) perfusion dynamics data, here illustrated by the depiction of a so-called "contrast agent curve" as routinely generated for breast MRI examinations, depicting the signal intensity change over time after the contrast agent administration in a graph 4.

The perfusion dynamics data can be given in the form of a heatmap for each pixel of an NCMDI 1-I used as input. The numerical value provided by the heatmap for each pixel may indicate a perfusion intensity at that pixel for a specific point in time. The ANN 2 may be configured such as to generated, based on its input data, one or more of such heatmaps, preferably a plurality of heatmaps, each depicting a different point in time.

The ANN 2 may also be configured to identify a region of interest within the NCMDI 1-I, or to receive a user command indicating a region of interest via a user interface. The perfusion dynamics data can then, alternatively or additionally, also be given in the form of a table or graph 4 showing the integrated perfusion intensity over said region of interest as a function of time.

The ANN 2 may thus be configured to generate, as its output:
- one or more perfusion images 3, preferably a plurality for different points in time;
- one or more heatmaps indicating perfusion intensity, preferably a plurality for different points in time;

As has been described in the foregoing, the input module 110 may be configured to receive a plurality of non-contrast medical diagnostic images, NCMDIs 1-i, depicting different points in time, PIT, and the ANN 2 may be configured to receive input data based on the plurality of NCMDIs 1-i.

The computing device 150 may be configured to implement a preprocessing module 120 configured to preprocess any or all of the NCMDIs 1-i that are received by the input module 110 in order to transform them into, or derive from them, the input data for the ANN 2. The preprocessing my comprise, for example, co-registration of the non-contrast medical diagnostic images, NCMDIs 1-i or the like.

The artificial neural network, ANN 2, may comprise at least one convolutional layer, and may preferably have a U-net type architecture, see for example Ronneberger et al.

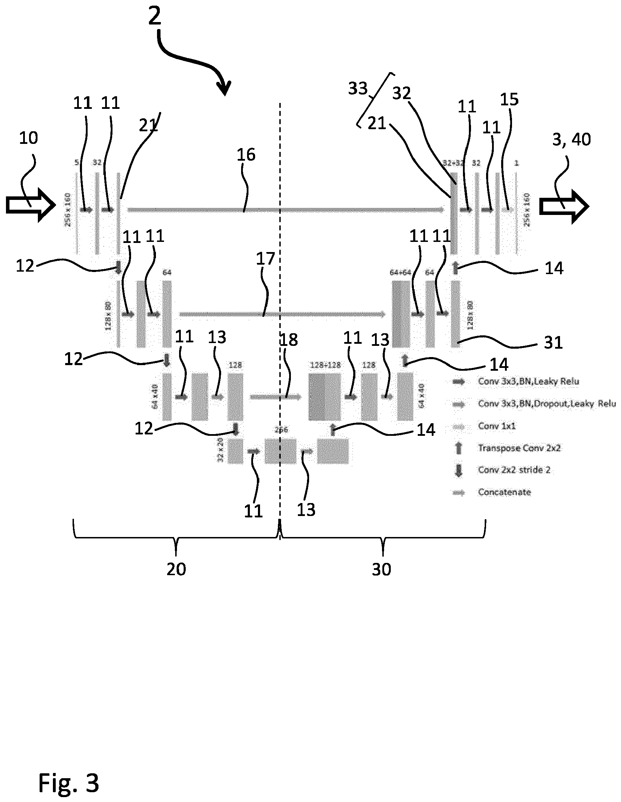

An exemplary realization is shown schematically in Fig. 3. As has been described in the foregoing, a U-net type network architecture comprises an encoder branch 20 and a decoder branch 30. In general, in the encoder branch 20, the number of features is reduced and the number of channels is increased, whereas in the decoder branch 30 in general the number of channels is decreased and the number of features is increased.

In the shown example, input data 10 consist of the five non-contrast medical diagnostic images, NCMDIs 1-i, here exemplarily given with 256x160 pixels. In Fig. 3, the vertically drawn numbers in the format "a x b" indicate the number of features arranged in a matrix with a rows and b columns. In the field of biomedical imaging, or with convolutional layers in general, the features may be identified with pixels, wherein the numerical value of each features equals the color or greyscale value of a corresponding pixel.

The horizontal numbers in Fig. 3 denote the number of channels. Since the input data 10 here consists of five NCMDIs 1-i, the initial number of channels is 5, wherein each channel corresponds to an NCMDI 1-i acquired at a different time. Since the NCMDIs 1-i are all taken (preferably) with the same imaging device and the same settings, the resolution and dimensions of the NCMDIs 1-i is the same so that each of the 5 channels has the same size of 256×160 pixels. The strips or rectangles in Fig. 3 correspond to the feature maps, and the arrows in between correspond to operations performed by layers of the ANN 2. Simply said, each arrow corresponds to a layer.

For the present example, the following types of layers may be used:
- convolutional layers 11 with a 3×3 kernel, batch normalization and a leaky ReLu activation function;
- convolutional layers 12 with a 2×2 kernel and a stride of 2;
- convolutional layers 13 with a 3×3 kernel, batch normalization, a dropout function (only during training) and a leaky ReLu activation function;
- transposed convolutional layers 14 (or: deconvolutional layers) with a 2×2 kernel; and
- a convolutional layer 15 with a 1×1 kernel,
wherein the layers are arranged as shown in Fig. 3. The numbers of features and channels of each feature map depends on the numbers of features and channels of the preceding feature map as well as on the nature of the layer in between, in particular, in the case of convolutional or deconvolutional layers, on the size of the convolutional kernels, on the number of convolutional kernels and on the stride applied. If the number of features is desired to be kept constant for a layer, the preceding feature map may be subjected to padding. In Fig. 3, the number of kernels applied by each layer can be inferred from the changes in the numbers of channels. Feature maps without explicit channel numbers shown have the same number of channels as their preceding feature maps.

As is characteristic for a U-net, there is not only the linear sequence of the result of the encoder branch 20 being fed into the decoder branch 30, but there are also additional shortcut connections 16, 17, 18 between the encoder branch 20 and the decoder branch 30. The shortcut connections 16, 17, 18 simply take the feature map (of the encoder branch 20) from which they originate and concatenate it with a feature map (of the decoder branch 30) at which they terminate.

For example, in the decoder branch 30, a transpose convolutional layer 14 is applied to a feature map 31 with 128×80 pixels and 64 channels, the result of which is a feature map 32 with 256×160 pixels and 32 channels. The first shortcut connection 16 takes a feature map 21 (the result of two convolutional layers 11 applied to the input data 10) which has 256×160 features and 32 channels from the encoder branch 20, and concatenates it with the feature map 32 so as to create a new feature map 33 with 256×160 features and 32+32 channels. This feature map 33 is then treated further by the remainder of the decoder branch 30, in the shown case by the subsequent application of two convolutional layers 11 and a convolutional layer 15. The same applies for the other shortcut connections 17, 18.

The result of the ANN architecture shown in Fig. 3 are the output data 40, in this case a feature map with 256×160 pixels and 1 channel, or, in other words: a greyscale image of the same dimensions as the non-contrast medical diagnostic images, NCMDI 1-i. This is the generated perfusion image 3.

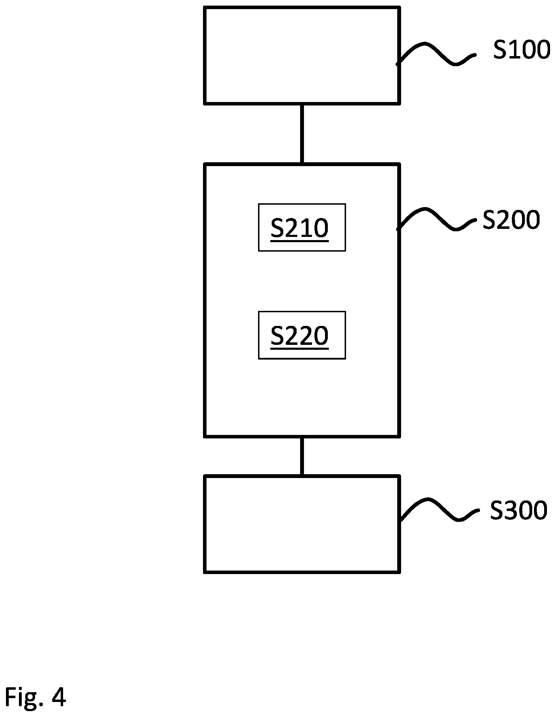

Fig. 4 shows a schematic flow diagram illustrating a method according to an embodiment of the second aspect of the present invention, i.e. a computer-implemented method for generating a perfusion image. The method as described in the following with respect to Fig. 4 may be performed using the apparatus according to any embodiment of the present invention, in particular the apparatus 100 of Fig. 1. The method may thus be adapted, modified, or refined based on any option, variant, modification, or refinement that has been described for the embodiments according to the first aspect of the present invention and vice versa.

In a step S100, at least one non-contrast medical diagnostic image, NCMDI 1-i, is received which shows, or depicts, living organic tissue. Again, the living organic tissue may be, for example, breast tissue, prostate issue and/or the like. A plurality of NCMDIs 1-i may be received. Preferably, these NCMDIs 1-i show the same patient and are taken at different points in time during the same examination session, for example at regular intervals within a time span of 5 minutes. The NCMDIs 1-i may be specifically non-contrast magnetic resonance imaging results, NCMRIR, of human breasts.

In a step S200, a perfusion image 3 is generated for the living organic tissue shown in the at least one non-contrast medical diagnostic image, NCMDI, 1-i. The perfusion image 3 is generated using an artificial neural network, ANN 2, trained and configured to receive input data 10 based on at least one of the received at least one medical diagnostic image, NCMDI 1-i. The artificial neural network, ANN 2, is trained and configured to generate the perfusion image 3 based on the input data 10, i.e. it receives the input data 10 at its input nodes and outputs an output based thereon, wherein the output comprises at least data which comprises, or indicates, or represents, the perfusion image 3. Step S200 may be performed in particular as has been described in the foregoing with respect to the computing device 150 and the artificial neural network, ANN 2. In step S200, additionally or even alternatively to the generating of the perfusion image 3, perfusion dynamics data 4 may be generated. Thus, step S200 may comprise generating S210 the perfusion image 3 and/or generating S220 the perfusion dynamic data 4.

In a step S300, at least the generated perfusion image 3 is output, for example as has been described with respect to the output module 190 in the foregoing.

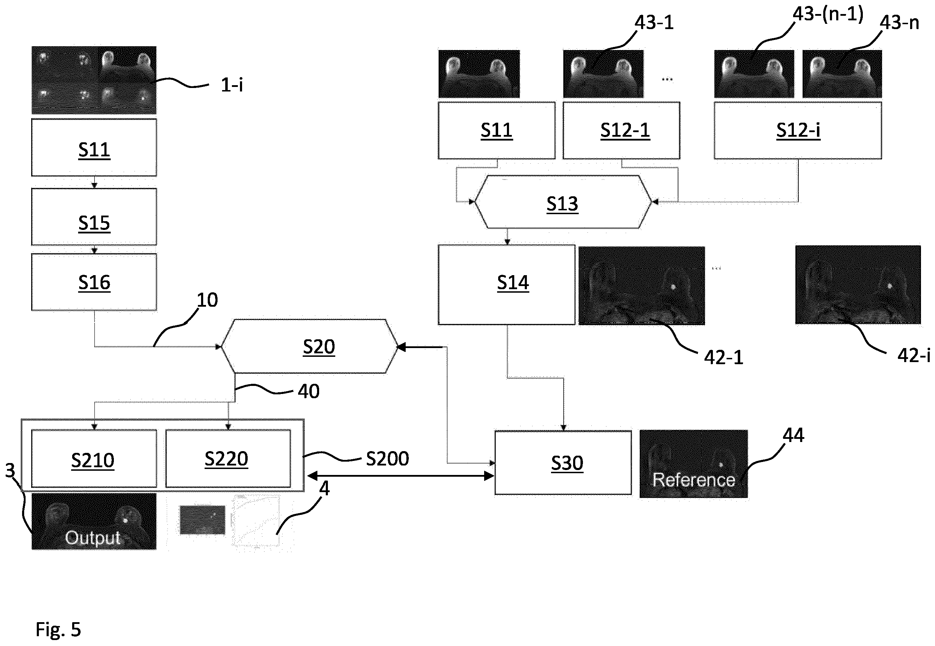

Fig. 5 shows a schematic flow diagram illustrating a method according to an embodiment of the third aspect of the present invention, i.e. a computer-implemented method for training an artificial neural network, ANN 2, for generating a perfusion image 3. The ANN 2 trained with this method may be used in the apparatus according to any embodiment of the present invention, in particular the apparatus 100 of Fig. 1. The ANN 2 trained with this method may be used in the method according to any embodiment of the second aspect of the present invention, in particular the method as illustrated with Fig. 4. The method may thus be adapted, modified, or refined based on any option, variant, modification, or refinement that has been described for the embodiments according to the first aspect of the present invention or to the second aspect of the present invention and vice versa.

In a step S10, a training set of medical diagnostic training image groups, MDTIG, is provided. Each medical diagnostic training image group, MDTIG, comprises at least:
- a non-contrast medical diagnostic image, NCMDI 1-i; and
- at least one subtraction image based on the NCMDI 42-i.

Step S10 may comprise sub-steps of, for example:
providing S11 a non-contrast medical diagnostic image, NCMDI 1-i, for an medical data training image group, MDTIG;
providing S12-i one or more contrast-enhanced medical diagnostic image, CEMDI 43-i;
preprocessing S13, the non-contrast medical diagnostic image, NCMDI 1-i and at least one of the one or more contrast-enhanced medical diagnostic image, CEMDI 43-i, wherein the preprocessing may comprise for example co-registration;
calculating S14 a subtraction image 42-i based on the NCMI 1-i and at least one CEMDI 43-i.

In other variants, subtraction images 42-i may be obtained from existing databases together with the NCMDIs 1-i and/or CEMDIs 43-i they are based on. Such databases may exist, for example, in picture archiving and communications systems, PACS, which are commonly used in hospitals and research institutes for receiving, storing, and providing medical imaging data.

Providing S10 the medical data training image groups, MDTIGs, may also comprise further sub-steps such as data augmentation S15, co-registration S16 of the non-contrast medical diagnostic images, NCMDIs 1-i, with each other and/or the like. Data augmentation S15 can include, for example, adding random noise, rotating the images, shifting the images and/or the like. For example, two images having content which is only rotated respectively to one another by 5° or so will easily be understood to show essentially the same information by a human. By contrast, for an artificial intelligence entity such as an artificial neural network, ANN, that commonly operates in a pixel-by-pixel manner, such images may look completely different. Data augmentation treats this problem as well as contributes to simply having more training data.

In a step S20, an artificial neural network, ANN 2, configured to receive, as input data 10, a non-contrast medical diagnostic image, NCMDI 1-i, and to generate S200, based on the input data 10, at least a perfusion image 3, is provided. This ANN 2 may have randomized initial parameters or, preferably, it may be an ANN 2 that has been pre-trained for a similar task on the same type of images.

In a step S30, the provided artificial neural network, ANN 2, is trained using the provided training set of medical diagnostic training image groups, MDTIG, with supervised learning. In the supervised learning, differences between the generated perfusion image 3 and at least one of the at least one subtraction image 42-i (which therefore acts as reference, or label 44) are penalized by a loss function. The loss function may be calculated pixel-by-pixel, wherein differences may be calculated according to any known similarity metric (or: difference metric). In some variants, pixel-wise algebraic loss functions such as mean squared error, MSE, loss functions or sum of squared errors, SSE, loss function may be employed.

The supervised training may be performed according to any method or variant known to the skilled person. For example, as has been described with respect to Fig. 3, dropout functions may be applied to selected layers during the training. The number of training epochs, the number of medical data training image groups, MDTIGs, in each epoch and so on will be selected by the skilled person based on the task at hand.

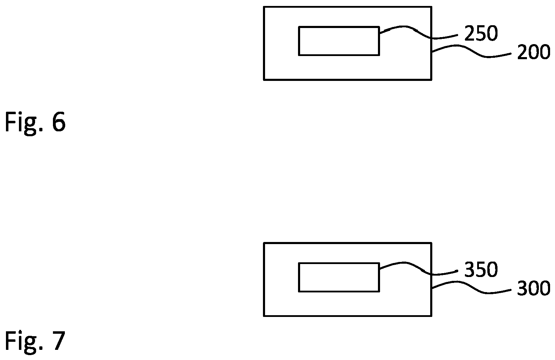

Fig. 6 shows a schematic block diagram illustrating a computer program product 200 according to an embodiment of the fourth aspect of the present invention. The computer program product 200 comprises executable program code 250 configured to, when executed, perform the method according to any embodiment of the second aspect of the present invention and/or the third aspect of the present invention, in particular as has been described with respect to the preceding figures.

Fig. 7 shows a schematic block diagram illustrating a non-transitory computer-readable data storage medium 300 according to an embodiment of the fifth aspect of the present invention. The data storage medium 300 comprises executable program code 350 configured to, when executed, perform the method according to any embodiment of the second aspect of the present invention and/or the third aspect of the present invention, in particular as has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick, or the like.

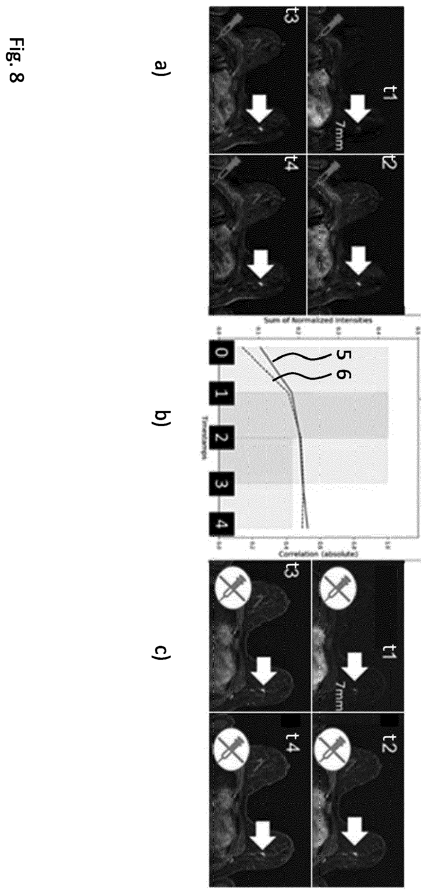

Fig. 8 shows comparison data between results of the present invention and results according to the prior art.

Fig. 8a) shows four different frames, each depicting a subtraction image 42-i at a different time point t1, t2, t3, t4. As indicated by the small syringe in the lower left corner of each frame, these subtraction images have been generated in a manner known in the prior art from an non-contrast medical diagnosis image, NCDMI 1-0, taken at a time point t0 in combination with contrast-enhanced medical diagnosis images, CEMDIs 43-1, 43-2..., 43-5, each taken at one of the time points t1, t2,..t5. The arrows in each image points to a region of interest in this particular patient.

Fig. 8 c) shows, results for perfusion images 4 for the same time points t1, t2, t3, t4, that have been generated using the apparatus 100 and method described herein, with input data for the ANN 2 based on the NCMDI 1-0. The comparison between Fig. 8a) and Fig. 8c), in particular with respect to the region of interest shows that the present invention works extraordinarily well without any contrast agent being actually administered to a patient (symbol of crossed-out syringe in Fig. 8c)).

Fig. 8b) shows a graph depicting total perfusion intensity (vertical axis) in the area of interest (at the tip of the arrow symbol) as a function of time steps t0, t1, t2, ... t4 (horizontal axis). In Fig. 8b), a measured intensity curve 6 corresponding to the subtraction images 42-i in Fig. 8a) is shown, as well as a predicted intensity curve 5 corresponding to the generated perfusion images 4 in Fig. 8c). As is evident, the correlation between the predicted intensity curve 5 and the measured intensity curve 6 is about 0.99.

In the foregoing detailed description, various features are grouped together in one or more examples or examples with the purpose of streamlining the disclosure. It is to be understood that the above description is intended to be illustrative, and not restrictive. It is intended to cover all alternatives, modifications, and equivalents. Many other examples will be apparent to one skilled in the art upon reviewing the above specification.

The embodiments were chosen and described in order to best explain the principles of the invention and its practical applications, to thereby enable others skilled in the art to best utilize the invention and various embodiments with various modifications as are suited to the particular use contemplated.

### List of reference signs

- 1-i: non-contrast medical diagnostic image, NCMDI
- 2: artificial neural network, ANN
- 3: perfusion image
- 4: perfusion dynamics data
- 5: predicted intensity curve
- 6: measured intensity curve
- 10: input data
- 11: convolutional layer
- 12: convolutional layer
- 13: convolutional layer
- 14: transposed convolutional layer
- 15: convolutional layer
- 16: shortcut connection
- 17: shortcut connection
- 18: shortcut connection
- 20: encoder branch
- 21: feature map
- 30: decoder branch
- 31: feature map
- 32: feature map
- 33: concatenated feature map
- 40: output data
- 42-i: subtraction image
- 43-i: contrast-enhanced medical diagnostic image, CEMDI
- 44: label
- 100: apparatus
- 110: input module
- 120: preprocessing module
- 150: computing device
- 190: output module
- 195: display device
- S10..S30: method steps

- S100.. S300: method steps

## Claims

1. An apparatus (100) for generating a perfusion image (3), comprising:
an input module (110) configured to receive (S100) at least one non-contrast medical diagnostic image, NCMDI (1-i), acquired from organic tissue;
a computing device (150) configured to implement an artificial neural network, ANN (2), which is trained and configured to receive input data (10) based on at least one of the received at least one non-contrast medical diagnostic image, NCMDI (1-i), and
to generate (S200), based on the input data (10), a perfusion image (3) for the organic tissue in the at least one non-contrast medical diagnostic image, NCMDI (1-i); and
an output module (190) configured to output (S300) at least the generated perfusion image (3).

2. The apparatus (100) of claim 1,
wherein at least one of the at least one non-contrast medical diagnostic image, NCMDI (1-i), is a non-contrast magnetic resonance imaging result, NCMRIR.

3. The apparatus (100) of claim 1 or claim 2,
wherein the organic tissue is breast tissue.

4. The apparatus (100) of any of claims 1 to 3,
where the computing module (150) is configured to generate (S200) a plurality of perfusion images (3) out of the NCMDI, specifically reflecting different points in time after a contrast agent administration to be provided to the output module (190)

5. The apparatus (100) of claim 4, wherein the artificial neural network, ANN (2), is further configured to generate (S210), based on the input data (10), in addition to the perfusion image (3) also perfusion dynamics data (4).

6. A computer-implemented method for generating a perfusion image, comprising steps of:
receiving (S100) at least one non-contrast medical diagnostic image, NCMDI (1-i), acquired from organic tissue;
generating (S200), using an artificial neural network, ANN (2), trained and configured to receive input data (10) based on at least one of the received at least one non-contrast medical diagnostic image, NCMDI (1-i), based on the input data (10), at least a perfusion image (3) for the organic tissue shown in the at least one non-contrast medical diagnostic image, NCMDI (1-i); and
outputting (S300) at least the generated perfusion image (3).

7. The method of claim 6,
wherein the non-contrast medical diagnostic image, NCMDI (1-i), is a non-contrast magnetic resonance imaging result, MCMRIR.

8. The method of claim 6 or claim 7,
wherein the organic tissue is breast tissue.

9. The method of any of claims 6 to 8,
further comprising generating (S220) based on the input data (10), perfusion dynamics data (4).

10. A computer-implemented method for training an artificial neural network (2) for generating a perfusion image (3), comprising steps of:
- providing (S10) a training set of medical diagnostic training image groups, MDTIG, wherein each medical diagnostic training image group, MDTIG, comprises at least:
- a non-contrast medical diagnostic image, NCMDI (1-i),
- at least one subtraction image (42-i) based on the NCMDI (1-i);
- providing (S20) an artificial neural network, ANN (2), configured to receive, as input data (10), a non-contrast medical diagnostic image, NCMDI (1-i), and to generate (S200), based on the input data (10), at least one perfusion image (3);
- training (S30) the provided artificial neural network, ANN (2), using the provided training set of medical diagnostic training image groups, MDTIG, using supervised learning while penalizing differences between the generated perfusion image (3) and at least one of the at least one subtraction image (42-i).

11. The method of claim 10,
wherein each medical data training image group, MDTIG, further comprises at least one contrast-enhanced medical diagnostic image, CEMDI (43-i); and
wherein the providing (S10) of the training set of MDTIGs comprises calculating (S14) the at least one subtraction image (42-i) of the MDTIG based on the non-contrast medical diagnostic image, NCMDI (1-i), and on the at least one contrast-enhanced medical diagnostic image, CEMDI (43-i), of the MDTIG.

12. The method of claim 11,
wherein each medical data training image group, MDTIG, comprises a plurality of non-contrast medical diagnostic images, NCMDIs (1-i); and
wherein the artificial neural network, ANN (2), is configured to receive, as input data (10), the plurality of NCMDIs (1-i) and to generate (S210) the perfusion image (3) for the supervised learning based on these input data.

13. The method of claim 12,
wherein each MDTIG further comprises a perfusion dynamics data label;
wherein the artificial neural network, ANN (2), is further configured to generate (S220), based on the input data (10), perfusion dynamics data (4); and
wherein training (S30) the ANN further comprises penalizing differences between the generated perfusion dynamics data (4) and the perfusion dynamics data label.

14. A computer program product (200) comprising executable program code (250) configured to, when executed, perform the method according to any of claims 6 to 13.

15. A non-transitory computer-readable data storage medium (300) comprising executable program code (350) configured to, when executed, perform the method according to any of claims 6 to 13.
